# EUROPEAN PATENT SPECIFICATION

(11) **EP 2 581 387 B1**
(45) Date of publication and mention of the grant of the patent: **16.09.2015**
(21) Application number: 11306314.3
(22) Date of filing: 11.10.2011
(51) Int. Cl.: C07K 16/10, A61K 39/395, A61P 31/18

(54) **An anti-HIV-1 Tat monoclonal antibody**
Monoklonaler Anti-HIV-1-Tat-Antikörper
Anticorps monoclonal tat anti-VIH-1

(43) Date of publication of application: 17.04.2013
(73) Proprietor: Université d'Aix-Marseille, 13284 Marseille (FR); INSERM (Institut National de la Santé et de la Recherche Médicale), 75654 Paris Cedex 13 (FR); Centre National de la Recherche Scientifique, 75016 Paris (FR)
(72) Inventor: Loret, Erwann, 13600 La Ciotat (FR); Baillat, Gilbert, 13011 Marseille (FR); Mediouni, Sonia, 13002 Marseille (FR); Watkins, Jennifer Daniele, Brighton, MA 02135-7245 (US); Pierres, Michel, 13016 Marseille (FR)
(74) Representative: Chajmowicz, Marion

(56) References cited:
- WO-A2-2006/088740
- OPI SANDRINE ET AL: "Tat HIV-1 primary and tertiary structures critical to immune response against non-homologous variants", JOURNAL OF BIOLOGICAL CHEMISTRY, THE AMERICAN SOCIETY OF BIOLOGICAL CHEMISTS, INC, US, vol. 277, no. 39, 27 September 2002 (2002-09-27), pages 35915-35919, XP002519492, ISSN: 0021-9258, DOI: 10.1074/JBC.M204393200
- CAMPBELL ET AL: "Tat mutations in an African cohort that do not prevent transactivation but change its immunogenic properties", VACCINE, ELSEVIER LTD, GB, vol. 25, no. 50, 22 October 2007 (2007-10-22), pages 8441-8447, XP022357908, ISSN: 0264-410X
- RACZ GABOR Z ET AL: "In vivo secretion of the mouse immunoglobulin G Fc fragment from rat submandibular glands.", THE JOURNAL OF GENE MEDICINE JUL 2009 LNKD- PUBMED:19424985, vol. 11, no. 7, July 2009 (2009-07), pages 580-587, XP002668919, ISSN: 1521-2254
- VALVATNE H ET AL: "A monoclonal antibody defines a novel HIV type 1 Tat domain involved in trans-cellular trans-activation.", AIDS RESEARCH AND HUMAN RETROVIRUSES 1 MAY 1996 LNKD- PUBMED:8743086, vol. 12, no. 7, 1 May 1996 (1996-05-01), pages 611-619, XP002668920, ISSN: 0889-2229
- SHEPHERD P S ET AL: "The design of the humanized antibody", 1 January 2000 (2000-01-01), MONOCLONAL ANTIBODIES: A PRACTICAL APPROACH, OXFORD UNIVERSITY PRESS, GB, PAGE(S) 58 - 66, XP008098266, ISBN: 978-0-19-963722-5

## Description

The present invention provides a neutralizing monoclonal antibody (mAb) against extracellular HIV-1- trans-activator of transcription protein (Tat).

### Technical background:

Antiviral treatment (HAART) against HIV-1 mainly target reverse transcriptase and protease, but HAART do not eradicate viruses in human even with the new class of antiviral agents targeting integrase. Passive immunization studies have demonstrated the protective role of monoclonal antibodies (mAb) in simian-human immunodeficiency virus (SHIV) challenge (Ruprecht RM., Methods Mol Biol 2009; 525: 559-66). All these studies have used antibodies targeting the Env protein of HIV-1 but were not efficient on human patients (Chen W, Dimitrov DS. AIDS Res Hum Retroviruses. 2011). Among other potential HIV-1 protein targets, Tat appears as the most attractive due to its extracellular functions (Campbell GR, Loret EP. Retrovirology 2009; 6: 50-62).

The HIV-1 trans-activator of transcription protein (Tat) has different roles for HIV-1. Tat increases HIV-1 genes transcription in infected cells while extra-cellular Tat can cross membranes and triggers the mitochondrial apoptosis of immune cells that should eliminate the virus (Campbell GR, Loret EP. Retrovirology 2009; 6: 50-62).

Tat has also been shown to modulate HIV spreading due to its interaction with gp120 at the cell surface (Marchio S, et al Blood 2005;105: 2802-11).

A Tat variant, called Tat Oyi has been identified, in the eighties, from a seropositive individual, in a remote area of Gabon, who did not develop AIDS. HIV-1 Oyi was cloned and has genes similar to usual HIV-1 strains except the *tat* gene, which has mutations never found in other Tat variants (Gregoire CJ, Loret EP. J Biol Chem, 1996; 271: 22641-6). Immunization of rabbits with Tat Oyi raised antibodies able to recognize Tat variants from the five main HIV-1 subtypes (Opi S, et al, J Biol Chem 2002; 277: 35915-9).

WO 2006/088740 discloses a monoclonal antibody against a linear epitope of HIV-1 TAT-protein. The epitope is referred to as a pan-epitope.

This is a need of a broadly neutralizing monoclonal antibody (mAb) against Tat with therapeutic potential.

### Summary of the invention:

Obtain broadly neutralizing mAb against Tat with therapeutic potential is important against HIV-1. The invention provides such mAb, named 7G12, which recognizes at least five subtypes of extracellular HIV-1 Tat (A, B, C, D, E). This mAb, or a fragment thereof, could be particularly useful as a therapeutic tool or for monitoring the quantity of Tat protein in a sample of a patient infected with a HIV-1.

The invention thus provides a monoclonal antibody (IgGl class) obtained from the hybridoma cell line deposited on September 29, 2011, at CNCM (Collection Nationale de Cultures de Microorganismes, Institut Pasteur, 25 rue du Docteur Roux, 75015 Paris), under registration number I-4535.

Any antibody that comprises the Fab fragment of the 7G12 antibody is encompassed, particularly fragments retaining neutralizing activity against extracellular HIV-1 Tat.

The disclosure further provides a monoclonal antibody that is substantially identical, preferably that shows an aminoacid sequence that shows more than 80%, preferably more than 85%, still preferably more than 90%, and still preferably more than 95% identity with the amino acid sequence of the antibody of 7G12 antibody.

Most preferably the antibodies of the invention exhibit a neutralizing activity against extracellular HIV-1- Tat.

Another subject of the invention is the hybridoma cell line deposited on September 29, 2011, at CNCM, under registration number I-4535.

The disclosure further provides a cell line that derives of, or is a progeny of, the above hybridoma cell line, and is still capable of producing a neutralizing monoclonal antibody (mAb) against extracellular HIV-1 trans-activator of transcription protein (Tat).

The invention further provides a method for detecting or determining the quantity of an extracellular Tat protein in a sample of a patient infected with HIV, by contacting the sample with an antibody of the invention.

It is further provided a pharmaceutical composition comprising the antibody in association with a pharmaceutically acceptable carrier.

The pharmaceutical composition is particularly useful as a therapeutic treatment against HIV-1 infection, wherein the HIV-1 infection may be from any HIV-1 subtype.

The invention also provides a method for obtaining the monoclonal antibody of claim 1, which method comprises the following steps: a) growing and amplifying the hydridoma I-4535 under suitable conditions that result in expression of the 7G12 antibody and b) isolating and purifying said antibody from the culture supernatant produced in step (a).

It is further described an *in vitro* or an *in vivo* method for the inhibition of HIV-1 viral replication in mammalian cells, which method comprises contacting the cells with the antibody as defined herein.

### Legends to the figures

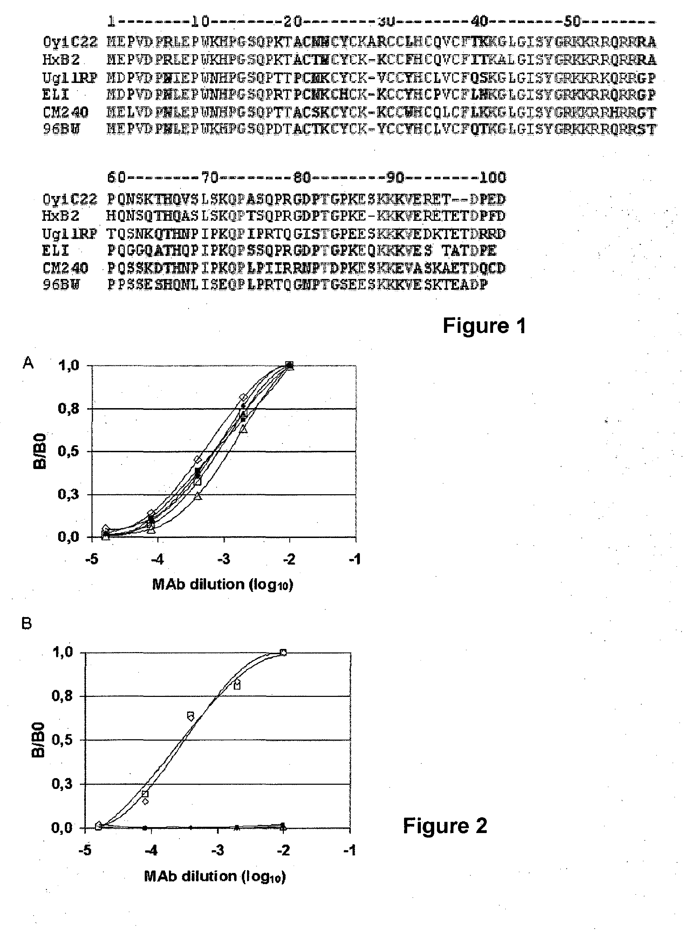

**Figure 1** shows a comparison of sequences of Tat Oyi and Tat variants representative of the five main HIV-1 subtypes (in grey, identities). The Oyi C22 sequence is shown as SEQ ID NO:1, the HxB2 sequence is shown as SEQ ID NO: 2, the UgllRP sequence is shown as SEQ ID NO:3, the ELI sequence is shown as SEQ ID N0:4, the CM2 40 sequence is shown as SEQ ID NO:5, and the 96BW sequence is shown as SEQ ID NO:6.
**Figures 2A and 2B** are graphs that show that MAb 7G12 cross-recognizes heterogonous Tat variants. Affinity curves of mAbs (A) 7G12 and (B) 27A8 were performed against different Tat variants: Oyi (white square), HxB2 (white diamond), UG11RP (white triangle), Eli (black square), CM240 (black diamond), 96Bw (black triangle). B/B0 corresponds to the OD measured for each Tat dilution divided by the maximal OD. The standard deviation measured is <0.02 (n=4).

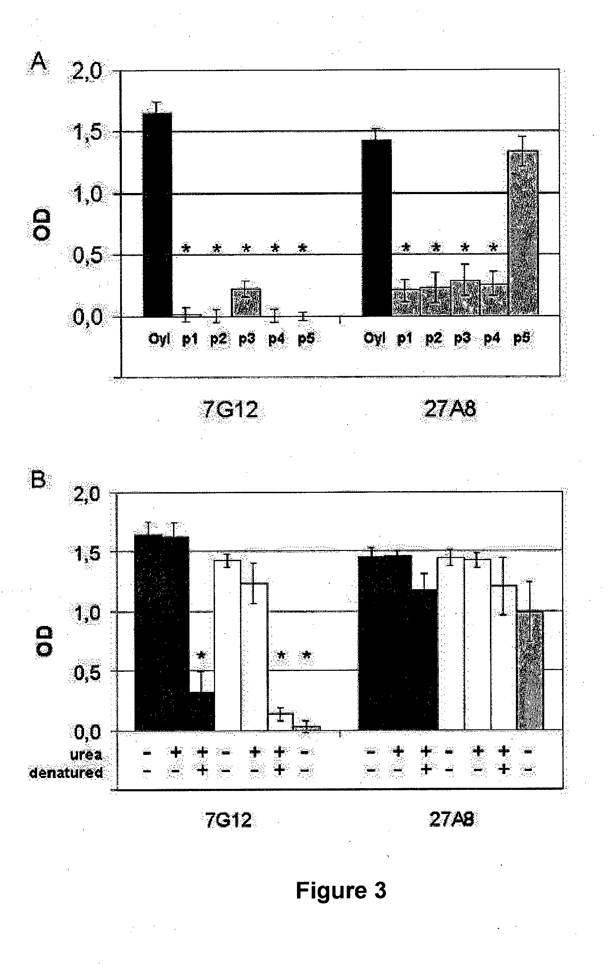

**Figures 3A and 3B** are graphs that show that MAb 7G12 recognizes a 3D epitope.
   Affinities (mean ± SD, n≥3) of mAbs 7G12 and 27A8 (1/1000) with (A) peptides 1 to 5 (p1 to p5) overlapping Tat Oyi sequence (grey bars) and (B) folded or denatured Tat Oyi (black bars) and Tat HxB2 (white bars) or a peptides pool (grey bars) were measured in ELISA. In bars graphs, statistical significant differences (P<0.01) between folded Tats and denatured proteins or peptides were indicated by (*). **Figure 3C** is a Western blot. Recognition of Tat Oyi by mAbs 7G12 and 27A8 (1/5000) was performed after Western blot analysis. Shown is a representative assay out of three experiments.

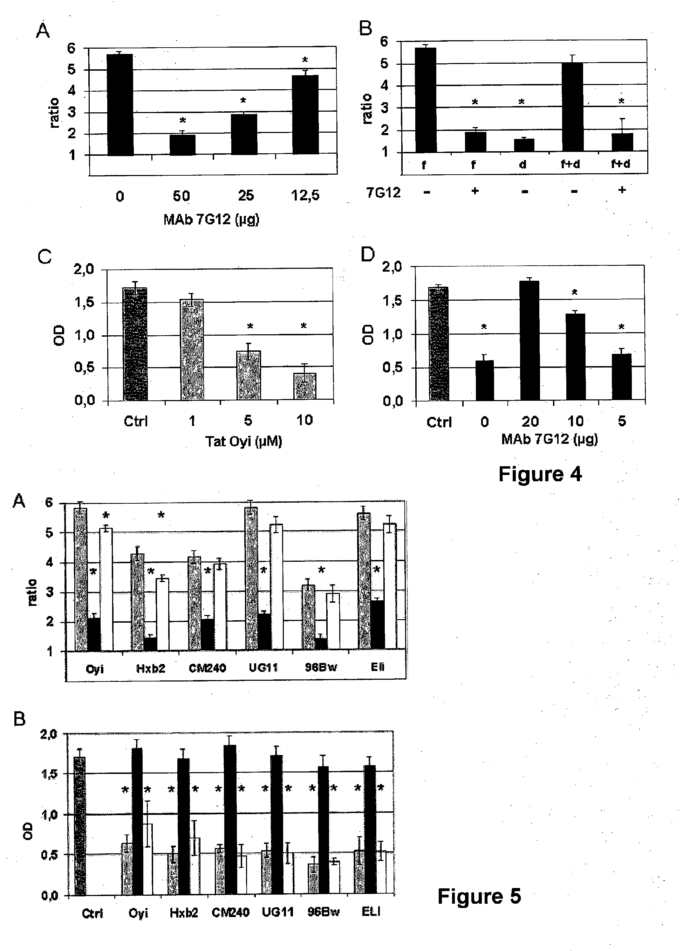

**Figures 4A to 4D** are graphs showing that MAb 7G12 neutralizes Tat Oyi biological activities. Transactivation ratio (mean ± SD, n≥3) was measured on HeLa P4 cells with (A) 0.5µM of Tat Oyi in presence of different concentrations of mAbs 7G12 (black bars) and with (B) 0.5 µM of folded (f) or denatured (d) Tat Oyi or a mix of both (f+d) in presence of 50µg of mAb 7G12. Jurkat cells proliferation (mean ± SD, n≥3) without Tat (Ctrl, dark grey bars) was compared with assays (C) in presence of different concentrations of Tat Oyi or (D) in presence of 5µM Tat Oyi and of different concentrations of mAb 7G12 (black bars). In bars graphs, statistical significant differences (P<0.05) were indicated by (*).
**Figures 5A and 5B** are graphs that show that MAb 7G12 cross-neutralizes also biological activities of other Tat variants. (A) Transactivation ratio (mean ± SD, n=3) of several Tat variants (0.5µM) were compared in absence (grey bars) or in presence of mAbs 7G12 (50µg, black bars) or 27A8 (50µg, white bars) in HeLa P4 cells. (B) Jurkat cells proliferation (mean ± SD, n=3) without Tat (Ctrl, dark grey bars) was compared with assays with 5µM of several Tat variants in absence (grey bars) or in presence of mAbs 7G12 (20µg, black bars) or 27A8 (20µg, white bars). In bars graphs, statistical significant differences (P<0.05) were indicated by (*).

### Detailed description of the invention

The inventors used immunogenic property of Tat Oyi protein to obtain a broadly neutralizing mAb, named 7G12, selected for its strong recognition of several genetically heterogonous Tat proteins (Tats) (Figures 1 and 2). They showed that mAb 7G12 recognizes a 3D epitope (Figure 3) and neutralizes activity of all Tat variants tested (Figures 4 and 5). Inhibition of Tat is observed with a molar ratio Tat/mAb 7G12 of 1/1 for transactivation and 2/1 for apoptosis.

Since the affinities of the antibody and the levels of neutralization were comparable for all Tats, the inventors then hypothesized that they could adopt a common structure, responsible for their biological activities and recognized by the mAb. The absence of recognition of Tat variants by mAb in ELISA and on Western blot after urea/heat degradation confirms also that there is a common 3D epitope in Tat variants.

The mAb could be used in a number of methods.

In particular it is useful for selecting neutralizing antibodies that bind the same 3D epitope. Also the antibody can be used in a competitive assay for screening antibodies, such as antibodies directed against folded Tats (Mediouni S, et al. Infect Disord Drug Targets ; 11: 57-63) that would be useful human therapeutic tools.

The mAb 7G12 itself may be used in a pharmaceutical composition, for passive immunization of HIV-1 infected patients, either alone or in combination with other mAbs, for instance anti-gp 120 antibodies.

### Definitions

The term "antibodies," as used herein, refers to monoclonal antibodies, as well as to fragments and derivatives of said antibodies unless otherwise stated or clearly contradicted by context. Depending on the type of constant domain in the heavy chains, full length antibodies typically are assigned to one of five major classes: IgA, IgD, IgE, IgG, and IgM. Several of these are further divided into subclasses or isotypes, such as IgG1, IgG2, IgG3, IgG4, and the like. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG and/or IgM are the preferred classes of antibodies employed in this invention because they are the most common antibodies in the physiological situation and because they are most easily made in a laboratory setting. The antibody 7G12 of the invention is an IgG1 immunoglobulin.

In another aspect, functional fragments and derivatives of the antibodies described herein can be prepared which have substantially similar antigen binding, specificity and/or activity, including, without limitation, Fab fragments, Fab'2 fragments, immunoadhesins, diabodies, camelized antibodies, Janusins, minibodies, CDRs, and ScFv fragments.

Unless otherwise specified, antibodies or bivalent fragments or derivatives thereof are monospecific, i.e., both "arms" of the antibodies, fragments, or derivatives bind the same antigen.

In yet another aspect, antibody derivatives comprising an antibody of the invention conjugated or covalently bound to a toxin, a radioelement, a detectable moiety (e.g., a fluor), or a solid support, can be prepared.

As used herein, the terms « isolated » and « purified » according to the invention refer to a level of purity that is achievable using current technology. The antibodies of the invention do not need to be absolutely pure (i.e., contain absolutely no molecules of other cellular macromolecules), but should be sufficiently pure so that one of ordinary skill in the art would recognize that they are no longer present in the environment in which they were originally found (i.e., the cellular medium). Thus, a purified or isolated molecule according to the present invention is one that has been removed from at least one other macromolecule present in the natural environment in which it was found.

More preferably, the antibodies of the invention are essentially purified and/or isolated, which means that the composition in which they are present is almost completely, or even absolutely, free of other macromolecules found in the environment in which the molecules of the invention are originally found. Isolation and purification thus do not occur by addition or removal of salts, solvents, or elements of the periodic table, but must include the removal of at least some macromolecules.

"Specific binding" or "specificity" refers to the ability of an antibody or other agent to detectably bind an epitope presented on an antigen, such as a Tat, while having relatively little detectable reactivity with other proteins or structures. Specificity can be relatively determined by binding or competitive binding assays, using, e.g., Biacore instruments, as described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules (in this case the specific antigen is a Tat).

"Selectivity" refers to the preferential binding of a protein to a particular target as opposed to one or more other biological molecules, structures, cells, tissues, etc. For example, selectivity can be determined by competitive ELISA or Biacore assays. The difference in affinity/avidity that marks selectivity can be any detectable preference (e.g., a ratio of more than 1:1.1, or more than about 1:5, if detectable, would be suitable, including 1:10, 1:100, 1:1000 or more). Unless otherwise specified, any quantitative data on "affinity" presented herein refers to measurements of bivalent (as opposed to monovalent) binding.

An "epitope" or "binding site" is an area or region on an antigen to which an antigen-binding peptide (such as an antibody) specifically binds. A protein epitope may comprise amino acid residues directly involved in the binding (also called immunodominant component of the epitope) and other amino acid residues, which are not directly involved in the binding, such as amino acid residues which are effectively blocked by the specifically antigen binding peptide (in other words, the amino acid residue is within the "footprint" of the specifically anti-gen binding peptide). The term epitope herein includes both types of amino acid binding sites in any particular region of a Tat that specifically binds to an anti-Tat antibody. Tats may comprise a number of different epitopes, which may include, without limitation, (1) linear peptide antigenic determinants, (2) conformational antigenic determinants which consist of one or more non-contiguous amino acids located near each other in a mature Tat conformation; and (3) post-translational antigenic determinants which consist, either in whole or part, of molecular structures covalently attached to a Tat, such as carbohydrate groups.

The term "binds to substantially the same epitope as" the monoclonal antibody 7G12 antibody means that an antibody "competes" with 7G12 antibody, generally for binding to HIV-1 Tat. Human, chimeric or humanized forms of the 7G12 antibody are encompassed. The term "substantially identical" in the context of two amino acid sequences means that the sequences, when optimally aligned, such as by the programs GAP or BESTFIT using default gap weights, share at least about 50, at least about 60, at least about 70, at least about 80, at least about 90, at least about 95, at least about 98, or at least about 99 percent sequence identity. In one embodiment, residue positions that are not identical differ by conservative amino acid substitutions. Sequence identity is typically measured using sequence analysis software. Protein analysis software matches similar sequences using measures of similarity assigned to various substitutions, deletions and other modifications, including conservative amino acid substitutions. For instance, the publicly available GCG software contains programs such as "Gap" and "BestFit" which can be used with default parameters to determine sequence homology or sequence identity between closely related polypeptides, such as homologous polypeptides from different species of organisms or between a wild-type protein and a mutated protein thereof. See, e.g., GCG Version 6.1. Polypeptide sequences can also be compared using FASTA, applying default or recommended parameters. A program in GCG Version 6.1., FASTA (e.g., FASTA2 and FASTA3) provides alignments and percent sequence identity of the regions of the best overlap between the query and search sequences (Pearson, Methods Enzymol. 1990;183:63-98; Pearson, Methods Mol. Biol. 2000;132:185-219). Another preferred algorithm when comparing a sequence to a database containing a large number of sequences from various organisms is the computer program BLAST, especially blastp, using default parameters. See, e.g., Altschul et al., J. Mol. Biol. 1990;215:403-410; Altschul et al., Nucleic Acids Res. 1997;25:3389-402 (1997).. "Corresponding" amino acid positions in two substantially identical amino acid sequences are those aligned by any of the protein analysis software mentioned herein, using default parameters.

### Production of antibody

The monoclonal antibody of the invention may be obtained from the hybridoma deposited as I-4535 at CNCM. Or it may be recombinantly produced by any method known to one of skill in the art, for example by recombination in a host cell, transformed with one or more vectors enabling the expression and/or secretion of the nucleotide sequences encoding the heavy chain or the light chain of the antibody. The vector generally contains a promoter, translation initiation and termination signals, and suitable transcriptional regulatory regions. It is stably maintained in the host cell and may optionally possess specific signals for secretion of the translated protein. These different components are selected and optimized by one of skill in the art according to the host cell used.

When the antibody is obtained from the hybridoma, the latter may be grown on a feeder layer of macrophages. The macrophages are preferably from littermates of the non-human mammal used to isolate splenocytes and are typically primed with incomplete Freund's adjuvant or the like several days be-fore plating the hybridomas. Fusion methods are described in Goding, "Monoclonal Antibodies: Principles and Practice," pp. 59-103 (Academic Press, 1986). The cells are allowed to grow in the selection media for sufficient time for colony formation and antibody production. This is usually between about 7 and about 14 days. The hybridoma colonies are then assayed for the production of antibodies. The assay is typically a colorimetric ELISA-type assay, although several other types of assays may be employed, including immunoprecipitation and radioimmunoassay, or FACS, Biacore, Scintillation-proximity assays (SPA), or other types of assays well known in the art. The wells containing antibodies of the desired specificity are examined to determine if one or more distinct hybridoma cell colonies are present. If more than one colony is present, the cells may be re-cloned and grown to ensure that only a single cell has given rise to the colony producing the desired antibody. Positive wells with a single apparent colony are typically re-cloned and re-assayed to insure that only one monoclonal antibody is being detected and produced.

In a preferred embodiment, the non-human animal used to produce antibodies according to applicable methods of the disclosure is a mammal, such as a rodent (e.g., mouse, rat, etc.), bovine, porcine, horse, rabbit, goat, sheep, etc.

Antibodies also may be produced transgenically through the generation of a chordate (such as a mammal or bird) or a plant that is transgenic for the immunoglobulin heavy and light chain sequences of interest and production of the antibody in a recoverable form therefrom (see, e.g., Ma et al., Nature Rev. Genetics 4:794-805 (2003); Nolke et al., Expert Opin Biol Ther. 2003 Oct;3(7):1153-62; Schillberg et al., Cell Mol Life Sci. 2003 Mar;60(3):433-45; Tekoah et al., Arch Biochem Biophys. 2004 Jun 15;426(2):266-78). In connection with the transgenic production in mammals, antibodies and other proteins can be produced in, and recovered from, the milk of goats, cows, or other mammals. See, e.g., US Patents 5,827,690, 5,756,687, 5,750,172, and 5,741,957. Antibodies also may be produced in the eggs of birds and recovered therefrom. See, e.g., Tini et al., Comp Biochem Physiol A Mol Integr Physiol. 2002 Mar;131(3):569-74 and US Patent 4,550,019.

Antibodies may also be produced by selection of combinatorial libraries of immunoglobulins, as disclosed for instance in Ward et al., Nature, 341 (1989) p. 544.

Other derivatives within the scope of this disclosure include functionalized antibodies, i.e., antibodies that are conjugated or covalently bound to a toxin, such as ricin, diphtheria toxin, abrin and Pseudomonas exotoxin, or a therapeutic radionuclide such as, e.g., 90Y or 131I; to a detectable moiety, such as a fluorescent moiety, a diagnostic radioisotope or an imaging agent; or to a solid support, such as agarose beads or the like. Methods for conjugation or covalent bonding of these other agents to antibodies are well known in the art.

### Diagnostic methods

The antibodies of the invention are useful to detect or quantify any HIV-1 Tat protein. In particular, they are of particular interest to monitor the concentration of an extracellular Tat protein in a sample of a patient infected with HIV. The sample may be any fluid or tissue extract. For instance it may be blood or serum.

The antibodies of the invention are useful as capture antibodies or detection antibodies in an immunoassay.

The term "capture antibody" is intended to mean an antibody or a part of an antibody, preferably attached to a solid phase, which is capable of retaining an antigen that is an extracellular HIV-1 Tat protein, present in a biological sample, by affinity binding.

The presence of the antigens in the biological sample is revealed by "detection means". As regards the detection of the antigen, the invention in particular provides detection using at least one "detection antibody". Such a detection antibody, which is labeled, is capable of binding to the captured antigen, by affinity binding, by recognizing an epitope site which is different from that recognized by the capture antibody or identical due to the presence of a repeat motif in the capsid. As regards the detection of the antibodies, use may in particular be made of labeled anti-immunoglobulin or anti-isotype antibodies, for example anti-immunoglobulins G.

The term "labeled" refers both to direct labeling (via enzymes, radioisotopes, fluorochromes, luminescent compounds, etc.) and to indirect labeling (for example via antibodies which are themselves labeled directly or using reagents consisting of a labeled "affinity pair", such as, but not exclusively, the pair labeled avidin-biotin, etc.).

In particular, the invention therefore provides a method for detecting, in vitro, an infection with HIV in a biological sample, which method comprises a) bringing the biological sample into contact with an antibody of the invention; b) incubating the mixture under conditions allowing the formation of antigen-antibody complexes; c) revealing the antigen-antibody complexes formed, which optionally uses a labeled detection antibody capable of binding to the Tat protein.

This immunoassay may be carried out according to various formats well known to those skilled in the art: in solid phase or in homogeneous phase; in one step or in two steps; in a double sandwich method (sandwich for the two antigen and antibody detections); or in an indirect method (for the antibody detection) combined with a sandwich method (for the antigen detection), by way of non limiting examples.

According to a preferred embodiment, the capture antibody is immobilized on a solid phase. By way of non limiting examples of solid phase, use may be made of microplates, in particular polystyrene microplates, such as those marketed by the company Nunc, Denmark. Use may also be made of solid particles or beads; paramagnetic beads, such as those provided by Dynal or Merck-Eurolab (France) or else test tubes made of polystyrene or polypropylene, etc.

An immunoassay format of the type sandwiched between two antibodies (capture and detection antibodies) is particularly advantageous for detecting the antigens present in the biological sample, whereas the antibodies can be revealed using a capture antigen and a labeled conjugate which attaches to the antibody (according to a format commonly referred to as "indirect format"), for example labeled protein A or a labeled anti-immunoglobulin or anti-isotype antibody. An immunoassay format for detecting the antigens by competition is also possible. Other modes of immunoassay can also be envisioned and are well known to those skilled in the art.

ELISA assays, radioimmunoassays, or any other detection technique, can be used to reveal the presence of the antigen-antibody complexes formed.

The detection of the presence of antigens in the biological sample can be completed by quantification, for example by measuring the signals emitted by the labels (color, luminescence, radioactivity, etc.), according to the standard techniques known to those skilled in the art.

The antibodies of the invention may be part of a kit to detect extracellular Tat protein in a sample, wherein the kit may further comprise detection means to reveal the antigen-antibody complex.

### Antibody Screening and Selection

The antibodies of this invention are "neutralizing", "blocking", or "inhibitory" antibodies, in the sense that they reduce, neutralize and/or block, at least partially and detectably, Tat functions, as transactivation and/or apoptosis activities, and/or HIV replication. This activity may be tested for instance as shown in the Experimental section.

The identification of one or more antibodies that bind(s) to substantially or essentially the same epitope as the monoclonal antibodies described herein can be readily determined using any one of variety of immunological screening assays in which antibody competition can be assessed. A number of such assays are routinely practiced and well known in the art (see, e.g., U.S. Pat. No. 5,660,827). It will be understood that actually determining the epitope to which an antibody described herein binds is not in any way required to identify an antibody that binds to the same or substantially the same epitope as the monoclonal antibody described herein.

For example, where the test antibodies to be examined are obtained from different source animals, or are even of a different Ig isotype, a simple competition assay may be employed in which the control antibody is mixed with the test antibody and then applied to a sample. Protocols based upon ELISAs, radioimmunoassays, Western blotting, and the use of BIACORE analysis are suitable for use in such simple competition studies.

The antibodies of the invention preferably show substantially the same affinity to any Tat protein as the 7G2 antibody. In particular, the antibodies are preferably selected when they show an affinity with a K_{D} value of about 2 to 20nM.

### Formulations

Another object of the invention therefore relates to a pharmaceutical composition comprising said antibody as active ingredient, in combination with one or more pharmaceutically acceptable excipients.

In the present description, pharmaceutically acceptable excipient shall be understood to mean a compound or a combination of compounds entering into a pharmaceutical composition which do not cause side reactions and which for example facilitate the administration of the active ingredient(s), increase the half-life and/or efficacy thereof in the body, increase the solubility thereof in solution or else improve the storage thereof. These pharmaceutically acceptable excipients are well known and will be adapted by one of skill in the art according to the nature and method of administration chosen.

Suitable excipients are, for example, water, saline, dextrose, glycerol, ethanol, or the like and combinations thereof. In addition, if desired, the composition may contain minor amounts of auxiliary substances such as wetting or emulsifying agents, or pH buffering agents.

The antibody may thus be obtained in purified form, i.e. in a form exhibiting a degree of purity of at least 80%, 85%, 90%, 95%, or greater. The degree of purity is defined relative to the other proteins present in the mixture which are considered to be contaminants. This degree is evaluated by colorimetry of an SDS-PAGE using coomassie blue. Densitometric measurement of the bands makes it possible to quantify the degree of purity. The degree of purity may also be measured by reverse-phase HPLC, by measuring the area of the various peaks.

Preferably the formulation is stored in liquid form, or in lyophilized form.

Buffering compounds may be used, for example in the form of carbonate, phosphate, citrate, acetate, borate, trimethamine [(2-amino-2-hydroxymethyl-1,-3-propanediol), TRIS], glycine and lysine (PDA Journal of Pharmaceutical Science and Technology, Vol. 51(4), 1997: Excipients and their use in injectable products (SANDEEP NEMA, R.J. WASHKUHN, R.J. BRENDEL, pp166-171)).

The addition of a surfactant of the nonionic polymer type, such as polysorbate 80 (Tween® 80) or a poloxamer of the type poloxamer 188 (Pluronic F68® or Lutrol F68®) is also advantageous.

In a preferred manner the pharmaceutical composition comprises from approximately 0.2 to approximately 5 g/L of antibody, preferably approximately 0.3 g/L of antibody.

Preferably, the antibody is administered by the systemic route, in particular by the intravenous route, intramuscular route, intradermal, intraperitoneal or subcutaneous route, or by the oral route. More preferably, the composition comprising the inventive antibodies is given in several administrations, spread out over time.

Antibodies may be conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkalene glycols or triglycerides: such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders and contain 10-95% of active ingredient, preferably 25-70%.

In many instances, it will be desirable to have multiple administrations of the antibody.

Generally, each dose may comprise between about 5 µg to about 1mg of the antibody, preferably between 10 to 200µg. Other dosage ranges may also be contemplated by one of skill in the art.

### Therapeutic indications

The present invention is also intended to cover the pharmaceutical compositions for use as a medicinal product, in particular for use in treating HIV infection.

The antibodies of the invention are neutralizing antibodies with respect to any HIV-1 subtype, in particular with respect to 2, 3, 4 or more HIV-1 subtypes.

In particular they inhibit the Tat Oyi transactivation and apoptosis induced activity.

In a preferred embodiment, the antibodies of the invention inhibit the Tat transactivation from two, three, four, five or more HIV-1 subtypes selected from Oyi, A, B, C, D, E, F, G, H, J, and K.

Most preferably, the antibodies of the invention inhibit the Tat transactivation from five HIV-1 subtypes (besides Oyi), preferably A, B, C, D, and E.

The present invention thus provides a method for treating an HIV infection in a patient, which method comprises administering an antibody of the invention to the patient.

The antibodies described herein are candidates of value for developing a treatment of a large number, or even all, of the individuals infected with HIV.

The antibodies may be used in a pharmaceutical composition, for passive immunization of HIV infected patients, either alone or in combination with other mAbs, for instance anti-gp120 antibodies. The antibodies of the invention are advantageously combined with an antiretroviral therapy, using inhibitors of HIV polymerase and/or HIV integrase.

A therapeutic effect is recognized if seropositive patients that have no antiviral treatment have a lower viremia and a rise of lymphocyte CD4 cells.

For example a therapeutic effect is recognized when 30 % of a cohort of at least 55 HIV seropositive patients maintain their viremia under 50 copies/ml after interruption of their HAART treatment for two months.

In patients with declared AIDS, such anti-Tat treatment could limit the incidence of certain pathological conditions such as Kaposi's sarcoma or neurological syndromes which appear to be linked to a direct action of the Tat protein following their secretion by HIV-infected cells.

In asymptomatic patients, the pharmaceutical composition of the invention is expected to delay the progression toward AIDS due to a lower viremia and a rise of lymphocyte CD4 cells. Such a treatment would allow the patient to restore an efficient immune cellular response against HIV infected cells, as appears to be not the case at the onset of the infection. In particular, the restoration of the activity of the cytotoxic T lymphocytes (CTL), NK cells and macrophages, but whose activity is inhibited by Tat. The treatment would have the consequence of allowing the patients to become at least non progressors.

The figures and examples illustrate the invention without limiting its scope.

### EXAMPLES:

### METHODS

### Tat Variants and peptides Synthesis

Tat Oyi was assembled in solid phase synthesis as previously described (Péloponèse et al. J Biol Chem 1999; 274: 11473-8). A Ser→Cys substitution at position 22 in Tat Oyi sequence (Fig. 1) allowed recovering biological activity of Tat Oyi and its use in neutralization assays with antibodies. Five peptides covering the full sequence with overlaps (1-22, 13-46, 38-72, 57-86 and 72-101) and respectively named peptide 1 to 5 were also synthesized. Other synthesized Tats correspond to clade A, predominant in Uganda (Ug11RP), clade D, predominant in DR Congo (Eli), recombinant clade CRF_AE, predominant in Thailand (CM240), clade C, predominant in South Africa (96Bw), and clade B, predominant in Europe and the Americas (HxB2) (Fig. 1). Purification and analysis were performed, as previously described (Watkins JD, et al. Retrovirology 2006; 3: 8-17). After lyophilisation, biological activity of Tat variants were checked by transactivation assays with HeLa P4 cells as previously described (Opi S, et al. J Biol Chem 2002; 277: 35915-9).

### Immunization and monoclonal antibody production

Four BALB/c mice were immunized with 10 µg synthetic Tat Oyi in 100 µl adjuvant of calcium phosphate (Brenntag Biosector, Denmark) by the subcutaneous route. Two weeks later, mice were boosted with the same preparation. Five weeks later, mice were boosted again with the same preparation by the intramuscular route. Three control mice were immunized also with the same adjuvant and protocol but without Tat protein. On day 45, mice were were euthanized and terminally bled. The spleenocytes were immediately separated to hybridize with myeloma cells as described (Fuller SA, et al. John Wiley and Sons (editor) New York; 1991. pp. 11.01-11.11.5). Supernatants of isolated hybridoma were screened by Enzyme Linked Immune-Sorbent Essay (ELISA) against Tat Oyi to identify producing clones. Then, they were sub-cloned by limiting dilutions (< 1.0 cell per well) twice and antibody positive clones were screened by ELISA against firstly Tat Oyi, then against Tats Ug11RP, ELI, CM240, 96BW and HxB2.

Previously, a similar immunization with Tat Oyi of four rats has been performed and a mAb (IgG1), named 27A8, has been selected on its strong recognition of Tat Oyi and was used as control.

Selected hybridomas were cultured and mAbs (IgG1) were purified by protein G chromatography (Roche Diagnostics, Germany) according to the manufacturer's instruction.

After purification, antibodies were dialysed against Hepes buffer 20mM, NaCl 120mM, pH 7.3 and concentrated at 1mg/ml.

### Detection of purified mAbs responses by ELISA

The 96-well plates were coated with 100 ng of folded or denatured Tats or peptides 1 to 5 (1 µg/ml in Phosphate buffer 100mM, pH 4.5) or peptides pool (each peptide: 1 µg/ml in Phosphate buffer 100mM, pH 4.5). Tats (10µg/ml) were denatured by heating at 90°C during 20 min in presence of urea 3M, then diluted 10 fold with cold Phosphate buffer 100mM, pH 4.5. To control urea effect, folded Tats could be coated also in presence to 0.3M urea. Following blocking with 5% skim milk and washing steps, plates were incubated with 100 µl of diluted purified mAb for 1 h at 37°C. After washing steps, 100 µl of HRP-conjugated anti-mouse or anti-rat IgG (Amersham, USA) were added and the plates were allowed to incubate for 1 h at 37°C. After washing steps, 100 µl of ABTS substrate (Roche Diagnostics, Germany) was added. The absorbance was measured at wavelength 405 nm at 1 h.

### Measurement association rate constants of mAb 7G12 with Tats in solution

An ELISA based method was used for measuring, in solution, antigen/antibody association rate constants (Hardy F, et al, J Immunol Methods 1997; 200: 155-9). Briefly, 96-well plates were coated with 100 ng of Tat Oyi in Phosphate buffer 100mM, pH 4.5 for overnight at 4°C. One well on four was left uncoated. Following washing steps, the plates were blocked with 5% skim milk in PBS for 1 h. The antibody (lµg/ml) and the Tat variants (0 to 0.4µg/ml) solutions were prepared in Hepes buffer 20mM, NaCl 120mM, pH 7.3 at twice the final concentration. At time zero, similar volumes of antibody and antigen solutions were mixed and immediately (time zero of the kinetic analysis), 100 µl were transferred to three coated wells and one uncoated well. Each five min, the wells were filled with a sample of mAb/Tat mixture. Each sample was incubated in the wells for 4.5 min and removed. At the end of the kinetic analysis, plates were washed and incubated with HRP-conjugated anti-mouse IgG. After washing steps, 100 µl of ABTS substrate was added. The absorbance was measured at wavelength 405 nm at 1h. Association and dissociation rate constants (kₒₙ and k_{off}) were then determined, allowing the calculation of equilibrium dissociation constant (KD).

### Western Blot

Tat proteins (100 ng) were subjected to SDS-PAGE (15%) under reducing conditions (urea 6M in Laemmli sample buffer at 96°C for 10 min). Tats were then electro-transferred to nitrocellulose membranes. After blocking with 5% skim milk, strips of the membrane were incubated for 1h with mAbs 7G12 or 27A8 at 1:2500. The secondary antibody was HRP-conjugated anti-mouse or anti-rat IgG diluted 1/1000 and bands were revealed with H₂O₂ 0.1%, diaminobenzidine tetra hydrochloride (Sigma, USA) as substrate.

### Neutralization of Tat transactivation

Neutralizations of Tat transactivation were performed with HIV LTR transfected HeLa cells and analyzed by monitoring the production of β-Galactosidase (β-Gal) in HeLa-P4 cells as previously described (Peloponese et al J Biol Chem 1999; 274: 11473-8). Lyophilized or denatured Tats were diluted at 5 µM (or 10µM) in phosphate buffer 100mM pH 4.5. Before adding to cells, Tat was diluted to 0.5µM with 400µl of DMEM with 0.1% Bovine Serum Albumin (BSA) (Sigma-Aldrich) and 0.01% protamine A (Sigma, USA) and described concentrations of antibodies. Volumes were adjusted with Hepes buffer 20mM, NaCl 120mM, pH7.3. When added together, denatured and folded Tat had a final concentration of 0.5µM each.

After 18h at 37°C, β-Gal production was measured with a β-Gal ELISA kit (Roche Diagnostics, Germany) according to manufacturer's recommendations. Transactivation ratio was the amount of β-Gal in presence of Tat divided by the amount without Tat.

### Methyl Thiazolyl Tetrazolium (MTT) assay with Jurkat T-Cells

Jurkat T-cells were cultivated in RPMI 1640 medium with glutamine supplemented with 10% (v/v) heat-inactivated foetal calf serum, 100 µg/ml streptomycin and 100 units/ml penicillin. 10⁵cells in 100µl of RPMI with 0.1% BSA and 0.01% protamine A were transferred in wells of 96-wells plates. Lyophilized Tats were diluted at 55 µM (or 65µM) in phosphate buffer 100mM pH 4.5. Cells were incubated 48h at 37 °C with the indicated amounts of Tats and mAbs. Control wells were adjusted with 10µl of phosphate buffer 100mM pH 4:5 and 20µl of Hepes buffer 20mM, NaCl 120mM, pH7.3. 0.5 mg of MTT

(InterChim, France) was added and incubated 5 h. Formazan crystals were dissolved in 200µl dimethyl sulphoxide and absorbance at 510 nm was measured.

### Statistical analysis

Statistical differences were analyzed by use of Mann-Whitney test. A P value < 0.05 was considered significant.

### RESULTS

### MAb 7G12 cross-recognizes Tat variants from the five main HIV-1 subtypes.

Mice were immunized with Tat Oyi and one mAb, named 7G12, was selected among 132 pre-screened clones for its broadly reactive immune response against a panel of Tat variants representatives of different HIV-1 clades (Fig. 1). To characterize the cross recognition, the affinities of mAb 7G12 for the different Tat variants were evaluated in ELISA (Fig. 2A). MAb 7G12 bound all Tats with a similar affinity. A control mAb called 27A8 bound only Tats Oyi and HxB2 with high affinities but not the others (Fig. 2B). In order to quantify mAb 7G12 affinities for Tat variants, an ELISA based method (Hardy F, et al. J Immunol Methods 1997; 200: 155-9) was used for measuring antigen/antibody association rate constants in solution. Antigen and antibody were mixed and, at different time intervals, aliquots were withdrawn to determine by ELISA the amount of free antibody. The disappearance of free antibody reflected the time course of the association reaction. Affinity constant obtained for Tat Oyi was high (KD =7 ± 0.4 nM). KD for Tats UG11RP, Eli, CM240 and 96BW were very similar (12 ± 0.5, 5.7 ± 0.1, 3.2 ±0.1 and 7.4 ± 0.3 nM respectively). These results confirmed ELISA affinity curves (Fig. 2A) and suggested that mAb 7G12 recognized an epitope common in all Tat variants tested. Interestingly, sequences comparison shows only a small percentage of potential continuous epitopes (> 5 amino acids) between the different Tats (Fig. 1).

### MAb 7G12 recognizes a 3D epitope

To map the epitopes recognized by mAbs 7G12 and 27A8, ELISA was carried out as previously described (Mediouni S, et al. Infect Disord Drug Targets ; 11: 57-63), using 100 ng of different overlapping peptides spanning the entire sequence of Tat Oyi (Fig. 3A). MAb 7G12 did not recognize peptides while mAb 27A8 recognized peptide 5 covering the C-terminal sequence (72 to 100). Thus, mAb 27A8 recognized a linear epitope in the C-terminal domain, conserved only between Tats Oyi and HxB2 (identity 83.3%) but not in others Tat variants. These data suggested that mAb 7G12 did not recognize a linear epitope but a 3D epitope on the surface of Tat Oyi that corresponds to a particular folding highly conserved in Tat variants.

To test this hypothesis, ELISA with mAbs 7G12 and 27A8 were performed against folded and denatured Tats Oyi and HxB2 (Fig. 3B). Urea used for complete denaturation did not modify the ELISA responses for both mAbs. MAb 7G12 did not recognize Tat variants following denaturation or a peptides pool, highlighting that this antibody was directed against a 3D epitope and not against a linear epitope. In contrast, mAb 27A8 recognized denatured Tat variants and the peptides pool, confirming that it was directed against a linear epitope. Moreover, mAb 7G12 was also unable to recognize Tat Oyi in Western blot analysis (Fig. 3C). In contrast, mAb 27A8 in the same dilution strongly recognized Tat Oyi.

### MAb 7G12 neutralizes Tat Oyi transactivation

The neutralizing effect of mAb 7G12 on transactivation activity of 0.5 µM Tat Oyi was monitored with LTR transfected HeLa cells (Fig. 4A). 50 µg of mAb 7G12 neutralized almost completely Tat Oyi transactivation activity. The neutralization potency of mAb 7G12 was correlated with the concentration of antibody. In our assays, the maximal neutralization was reached for 0.33 nmoles of mAb against 0.25 nmoles of Tat, suggesting a molar-molar association.

To precise if the neutralizing effect of mAb 7G12 was due to recognition of a 3D epitope, transactivation activity was examined using 0.5 µM of folded and denatured Tat Oyi (Fig. 4B). Denatured Tat Oyi had lost the transactivation activity but did not prevent the biological effect of folded Tat when both were added together. Interestingly, unfolded Tat did not decrease the neutralizing effect of 50 µg of mAb 7G12 when folded and denatured Tat were in competition. This data showed that the activity of Tat Oyi in solution was conformation dependent and blocked by the mAb 7G12 which recognized a 3D epitope.

### MAb 7G12 inhibits Tat Oyi-induced apoptosis in lymphocytes

Tat can also cross membranes and trigger mitochondrial apoptosis (De Mareuil J, et al. Retrovirology 2005; 2: 5-15). Viability of Jurkat lymphocytes cells was monitored in presence of Tat Oyi, using a tetrazolium salts based colorimetric assay (Fig. 4C). 10 µM of Tat Oyi almost completely inhibited Jurkat cells proliferation. This inhibition was correlated with the concentration of Tat Oyi up to 1µM.

The neutralizing effect of mAb 7G12 on proliferation inhibition induced by Tat Oyi was evaluated (Fig. 4D). 20 µg of mAb 7G12 completely reversed the inhibition of 5 µM of Tat Oyi, and neutralization was observed up to 5 µg of mAb 7G12.

### MAb 7G12 cross-neutralizes also biological activities of other Tat variants

Cross-neutralizing activity of 50 µg of mAb 7G12 was evaluated using the transactivation assay with HeLa P4 cells and 0.5 µM of various HIV-1 subtypes including A, B, C, D, and the recombinant form A_E. MAb 7G12 was compared to mAb 27A8 (Fig. 5A). MAb 7G12 inhibited the transactivation activity of all Tat variants tested as strongly as with Tat Oyi, its immunogenic target. Indeed, mAb 7G12 inhibited 61.9%, 66.3%, 57.1%, 53.2% and 50.8% of transactivation activities of UG11RP, HxB2, 96Bw Eli and CM240 variants respectively, compared to 63.7% observed with Tat Oyi. In contrast, mAb 27A8 showed a low inhibitory effect only for Tat Oyi and Tat HxB2 (12 % and 19.4% respectively) and no significant inhibition with others Tats.

Neutralizing effects of mAbs 7G12 and 27A8 on proliferation inhibition induced by 5 µM of Tat variants were also examined (Fig. 5B). MAb 7G12, but not mAb 27A8, completely reversed the proliferation inhibition induced by the different Tats. Thus, mAb 7G12 neutralized the biological activities of the different Tat variants tested with a similar efficiency, suggesting that the conformational folding recognized by antibody was conserved in these variants and is related to the Tat activity.

## Claims

1. A monoclonal antibody that is obtained or obtainable from the hybridoma cell line deposited on September 29, 2011, at CNCM, under registration number I-4535.

2. A monoclonal antibody fragment, which comprises the Fab fragment of the antibody of claim 1.

3. A monoclonal antibody that comprises the Fab fragment of the antibody of claim 1.

4. The monoclonal antibody of claim 3 that exhibits a neutralizing activity against extracellular HIV-1- Tat.

5. A hybridoma cell line deposited on September 29, 2011, at CNCM, under registration number I-4535.

6. A pharmaceutical composition comprising the antibody or antibody fragment of any of claims 1 to 4, in association with a pharmaceutically acceptable carrier.

7. The pharmaceutical composition of claim 6, for use in treating HIV-1 infection.

8. The pharmaceutical composition for use of claim 7, wherein the HIV-1 infection is from any HIV-1 subtype.

9. A method for obtaining the monoclonal antibody of claim 1, which method comprises the following steps: a) growing and amplifying the hydridoma 1-4535 deposited with CNCM on September 29, 2011 under suitable conditions that result in expression of mAb 7G12; and b) isolating and purifying the 7G12 mAb from the culture supernatant produced in step (a).

10. An *in vitro* method for the inhibition of HIV-1 viral replication in mammalian cells, which method comprises contacting the cells with the antibody or antibody fragment of any of claims 1 to 4.

11. An *in vitro* method for detecting or determining the quantity of an extracellular Tat protein in a sample of a patient infected with HIV-1, by contacting the sample the antibody or antibody fragment of any of claims 1 to 4.

## Patentansprüche

1. Ein monoclonaler Antikörper, der erhalten wird oder erhältlich ist von der Hybrodomzelllinie, die am 29. September 2011 bei CNCM unter der Registriernummer I-4535 hinterlegt wurde.

2. Ein monoclonales Antikörperfragment, das das Fab-Fragment des Antikörpers nach Anspruch 1 umfasst.

3. Ein monoclonaler Antikörper, der das Fab-Fragment des Antikörpers nach Anspruch 1 umfasst.

4. Der monoclonale Antikörper nach Anspruch 3, der eine neutralisierende Wirkung gegen extrazelluläres HIV-1-Tat aufweist.

5. Eine Hybrodomzelllinie, die am 29. September 2011 bei CNCM unter der Registriernummer I-4535 hinterlegt wurde.

6. Eine pharmazeutische Zusammensetzung umfassend den Antikörper oder das Antikörperfragment nach einem der Ansprüche 1 bis 4 in Verbindung mit einem pharmazeutisch verträglichen Träger.

7. Die pharmazeutische Zusammensetzung nach Anspruch 6 zur Verwendung bei der Behandlung einer HIV-1 Infektion.

8. Die pharmazeutische Zusammensetzung zur Verwendung nach Anspruch 7, wobei die HIV-1 Infektion von irgendeinem HIV-1 Subtypen ist.

9. Ein Verfahren zum Erhalten des monoclonalen Antikörpers nach Anspruch 1, das Verfahren umfasst die folgenden Schritte: a) Wachsen und Amplifizieren des Hybrodoms I-4535, das bei CNCM am 29. September 2011 hinterlegt wurde, unter geeigneten Bedingungen, die zur Expression von mAb 7G12 führen; und b) Isolieren und Aufreinigen des 7G12 mAb aus dem Kulturüberstand, der in Schritt (a) erzeugt wurde.

10. Ein *in vitro* Verfahren zur Inhibierung von HIV-1 viraler Replikation in Säugetierzellen, das Verfahren umfasst das Inkontaktbringen der Zellen mit dem Antikörper oder Antikörperfragment nach einem der Ansprüche 1 bis 4.

11. Ein *in vitro* Verfahren zum Nachweis oder zur Bestimmung der Menge an einem extrazellulären Tat-Protein in einer Probe eines Patienten, der mit HIV-1 infiziert ist, durch Inkontaktbringen der Probe mit dem Antikörper oder Antikörperfragment nach einem der Ansprüche 1 bis 4.

## Revendications

1. Anticorps monoclonal obtenu ou pouvant être obtenu à partir d'une lignée cellulaire d'hybridome déposée le 29 septembre 2011, à la CNCM, sous le numéro d'enregistrement I-4535.

2. Fragment d'anticorps monoclonal, comprenant le fragment Fab de l'anticorps selon la revendication 1.

3. Anticorps monoclonal, comprenant le fragment Fab de l'anticorps selon la revendication 1.

4. Anticorps monoclonal selon la revendication 3, qui présente une activité de neutralisation contre la protéine extracellulaire Tat du VIH-1.

5. Lignée cellulaire d'hybridome déposée le 29 septembre 2011, à la CNCM, sous le numéro d'enregistrement I-4535.

6. Composition pharmaceutique comprenant l'anticorps ou le fragment d'anticorps selon l'une quelconque des revendications 1 à 4, en association avec un véhicule pharmaceutiquement acceptable.

7. Composition pharmaceutique selon la revendication 6, pour une utilisation dans le traitement de l'infection au VIH-1.

8. Composition pharmaceutique pour une utilisation selon la revendication 7, dans laquelle l'infection au VIH-1 provient de n'importe quel sous-type de VIH-1.

9. Méthode d'obtention de l'anticorps monoclonal selon la revendication 1, ladite méthode comprenant les étapes suivantes : a) croissance et amplification de l'hybridome I-4535 déposé à la CNCM le 29 septembre 2011 dans des conditions adaptées qui conduisent à l'expression du mAb 7G12 ; et b) isolement et purification du mAb 7G12 à partir du surnageant de culture produit à l'étape (a).

10. Méthode *in vitro* d'inhibition de la réplication virale du VIH-1 dans les cellules de mammifère, ladite méthode comprenant la mise en contact des cellules avec l'anticorps ou le fragment d'anticorps selon l'une quelconque des revendications 1 à 4.

11. Méthode *in vitro* de détection ou de détermination de la quantité d'une protéine Tat extracellulaire dans un échantillon d'un patient infecté par le VIH-1, par la mise en contact de l'échantillon avec l'anticorps ou le fragment d'anticorps selon l'une quelconque des revendications 1 à 4.


### SEQUENCE LISTING

<110> Université de la Méditerranée
<120> An anti-HIV1 Tat monoclonal antibody
<130> B1240
<160> 6
<170> PatentIn version 3.3
<210> 1
   <211> 100
   <212> PRT
   <213> Human immunodeficiency virus type 1
<400> 1
   Met Glu Pro Val Asp Pro Arg Leu Glu Pro Trp Lys His Pro Gly Ser
   1               5                   10                  15
   Gln Pro Lys Thr Ala Cys Asn Asn Cys Tyr Cys Lys Ala Arg Cys Cys
               20                  25                  30
   Leu His Cys Gln Val Cys Phe Thr Lys Lys Gly Leu Gly Ile Ser Tyr
           35                  40                  45
   Gly Arg Lys Lys Arg Arg Gln Arg Arg Arg Ala Pro Gln Asn Ser Lys
       50                  55                  60
   Thr His Gln Val Ser Leu Ser Lys Gln Pro Ala Ser Gln Pro Arg Gly
   65                  70                  75                  80
   Asp Pro Thr Gly Pro Lys Glu Ser Lys Lys Lys Val Glu Arg Glu Thr
                   85                  90                  95
   Asp Pro Glu Asp
               100
<210> 2
   <211> 100
   <212> PRT
   <213> Human immunodeficiency virus type 1
<400> 2
   Met Glu Pro Val Asp Pro Arg Leu Glu Pro Trp Lys His Pro Gly Ser
   1               5                   10                  15
   Gln Pro Lys Thr Ala Cys Thr Asn Cys Tyr Cys Lys Lys Cys Cys Phe
               20                  25                  30
   His Cys Gln Val Cys Phe Ile Thr Lys Gly Leu Gly Ile Ser Tyr Gly
           35                  40                  45
   Arg Lys Lys Arg Arg Gln Arg Arg Arg Ala His Gln Asn Ser Ser Thr
       50                  55                  60
   His Gln Ala Ser Leu Ser Lys Gln Pro Thr Ser Gln Pro Arg Gly Asp
   65                  70                  75                  80
   Pro Thr Gly Pro Lys Glu Lys Lys Lys Val Glu Arg Glu Thr Glu Thr
                   85                  90                  95
   Asp Pro Glu Asp
               100
<210> 3
   <211> 101
   <212> PRT
   <213> Human immunodeficiency virus type 1
<400> 3
   Met Asp Pro Val Asp Pro Asn Ile Glu Pro Trp Asn His Pro Gly Ser
   1               5                   10                  15
   Gln Pro Thr Thr Pro Cys Asn Lys Cys Tyr Cys Lys Val Cys Cys Tyr
               20                  25                  30
   His Cys Leu Val Cys Phe Gln Ser Lys Gly Leu Gly Ile Ser Tyr Gly
           35                  40                  45
   Arg Lys Lys Arg Lys Gln Arg Arg Gly Pro Thr Gln Ser Asn Lys Gln
       50                  55                  60
   Thr His Asn Pro Ile Pro Lys Gln Pro Ile Pro Arg Thr Gln Gly Ile
   65                  70                  75                  80
   Ser Thr Gly Pro Glu Glu Ser Lys Lys Lys Val Glu Asp Lys Thr Glu
                   85                  90                  95
   Thr Asp Arg Arg Asp
               100
<210> 4
   <211> 99
   <212> PRT
   <213> Human immunodeficiency virus type 1
<400> 4
   Met Asp Pro Val Asp Pro Asn Leu Glu Pro Trp Asn His Pro Gly Cys
   1               5                   10                  15
   Gln Pro Arg Thr Pro Cys Asn Lys Cys His Cys Lys Lys Cys Cys Tyr
               20                  25                  30
   His Cys Pro Val Cys Phe Leu Asn Lys Gly Leu Gly Ile Ser Tyr Gly
           35                  40                  45
   Arg Lys Lys Arg Arg Gln Arg Arg Gly Pro Pro Gln Gly Gly Gln Ala
       50                  55                  60
   Thr His Gln Pro Ile Pro Lys Gln Pro Ser Ser Gln Pro Arg Gly Asp
   65                  70                  75                  80
   Pro Thr Gly Pro Gln Glu Gln Lys Lys Lys Val Glu Ser Thr Ala Thr
                   85                  90                  95
   Asp Pro Glu
<210> 5
   <211> 101
   <212> PRT
   <213> Human immunodeficiency virus type 1
<400> 5
   Met Glu Leu Val Asp Pro Asn Leu Glu Pro Trp Asn His Pro Gly Ser
   1               5                   10                  15
   Gln Pro Thr Thr Ala Cys Ser Lys Cys Tyr Cys Lys Lys Cys Cys Trp
               20                  25                  30
   His Cys Gln Leu Cys Phe Leu Lys Lys Gly Leu Gly Ile Ser Tyr Gly
           35                  40                  45
   Arg Lys Lys Arg Arg His Arg Arg Gly Thr Pro Gln Ser Ser Lys Asp
       50                  55                  60
   Thr His Asn Pro Ile Pro Lys Gln Pro Leu Pro Ile Ile Arg Arg Asn
   65                  70                  75                  80
   Pro Thr Asp Pro Lys Glu Ser Lys Lys Glu Val Ala Ser Lys Ala Glu
                   85                  90                  95
   Thr Asp Gln Cys Asp
               100
<210> 6
   <211> 102
   <212> PRT
   <213> Human immunodeficiency virus type 1
<400> 6
   Met Glu Pro Val Asp Pro Asn Leu Glu Pro Trp Lys His Pro Gly Ser
   1               5                   10                  15
   Gln Pro Asp Thr Ala Cys Thr Lys Cys Tyr Cys Lys Tyr Cys Cys Tyr
               20                  25                  30
   His Cys Asn Leu Leu Val Cys Phe Gln Thr Lys Gly Leu Gly Ile Ser
           35                  40                  45
   Tyr Gly Arg Lys Lys Arg Arg Gln Arg Arg Ser Thr Pro Pro Ser Ser
       50                  55                  60
   Glu Ser His Gln Asn Leu Ile Ser Ser Glu Gln Pro Leu Pro Arg Thr
   65                  70                  75                  80
   Gln Gly Asn Pro Thr Gly Ser Glu Glu Ser Lys Lys Lys Val Glu Ser
                   85                  90                  95
   Lys Thr Glu Ala Asp Pro
               100